# EUROPÄISCHE PATENTSCHRIFT

(11) **EP 3 289 056 B1**
(45) Veröffentlichungstag und Bekanntmachung des Hinweises auf die Patenterteilung: **09.10.2019**
(21) Anmeldenummer: 16716213.0
(22) Anmeldetag: 14.04.2016
(51) Int. Cl.: C11D 1/92, C11D 1/94

(54) **VERWENDUNG VON SULFOBETAINHALTIGEN WASCH- UND REINIGUNGSMITTELN**
USE OF SULFOBETAINE-CONTAINING DETERGENTS AND CLEANING AGENTS
UTILISATION DES PRODUITS DE LAVAGE ET DE NETTOYAGE CONTENANT DE LA SULFOBÉTAÏNE

(30) Priorität: 28.04.2015 DE 102015207735
(43) Veröffentlichungstag der Anmeldung: 07.03.2018
(73) Patentinhaber: Henkel AG & Co. KGaA, 40589 Düsseldorf (DE)
(72) Erfinder: HELLMUTH, Hendrik, 64297 Darmstadt (DE); GERKE, Thomas, 40627 Düsseldorf (DE); BODE, Nicole, 40627 Düsseldorf (DE); STROTZ, Michael, 50969 Köln (DE); DREJA, Michael, 41469 Neuss (DE)
(86) Internationale Anmeldenummer: PCT/EP2016/058169
(87) Internationale Veröffentlichungsnummer: WO 2016/173848

(56) Entgegenhaltungen:
- DE-A1- 2 361 080
- DE-B- 1 018 421
- US-A- 4 671 894
- US-A- 5 336 445
- STASZAK K ET AL: "Synthesis and interfacial activity of novel sulfobetaines in aqueous solutions", TENSIDE SURFACTANTS DETERGENTS, CARL HANSER VERLAG, Bd. 50, Nr. 1, 1. Januar 2013 (2013-01-01) , Seiten 45-51, XP008180576, ISSN: 0932-3414, DOI: 10.3139/113.110233 in der Anmeldung erwähnt

## Beschreibung

Die vorliegende Erfindung betrifft die Verwendung von Morpholinderivaten der allgemeinen Formel (I) zur Verbesserung der Reinigungsleistung von Wasch- oder Reinigungsmitteln gegenüber öl- und/oder fetthaltigen Anschmutzungen beim Waschen von Textilien oder Reinigen harter Oberflächen in insbesondere builderhaltiger wässriger Flüssigkeit.

Die Entfernung von auf Textilien befindlichen Anschmutzungen ist vorrangiges Ziel des Textilwaschvorgangs. Ebenso ist die Entfernung von auf harten Oberflächen, wie Geschirr, Glas, Fliesen oder Haushaltsarbeitsflächen, befindlichen Anschmutzungen vorrangiges Ziel dort entsprechend angewendeter Reinigungsvorgänge. Dabei eingesetzte Wasch- oder Reinigungsmittel enthalten zu dem genannten Zweck Tenside und in der Regel weitere Inhaltsstoffe wie Bleichmittel oder Enzyme, welche in der Lage sind, Schmutz von der textilen Oberfläche oder der harten Oberfläche abzulösen oder Schmutzbestandteile chemisch zu modifizieren, beispielsweise durch Oxidation oder enzymatischen Abbau, so dass sie sich leichter von der Oberfläche ablösen lassen. Die weitere Verbesserung des Reinigungsergebnisses ist Ziel mannigfaltiger Anstrengungen.

Aus der Patentanmeldung DE 1 018 421 B ist ein Verfahren zur Herstellung von oberflächenaktiven Sulfobetainen aus tertiären Aminen und Sultonen bekannt. Die Patentanmeldung DE 2 361 080 A1 offenbart Shampoos, die polyethoxylierte anionische Tenside und zwitterionische oberflächenaktive Substanzen enthalten. Die Patentschrift US 4 671 894 betrift flüssige Geschirrspülmittel, die Alkylbenzolsulfonat und/oder Alkylsulfat, 3- bis 12-fach ethoxylierten C₆₋₁₃-Alkohol und zwitterionisches Tensid mit C₁₀₋₁₆-Alkylgruppen enthalten. Aus der patentschrift US 5 336 445 sind flüssige Reinigungsmittel für harte Oberflächen bekannt, die Beta-Amionoalkanole und Tenside enthalten, worunter auch Betaintenside sein können.

Überraschenderweise wurde gefunden, dass der Einsatz bestimmter amphoterer Morpholinderivate zur Verbesserung des Wasch- und Reinigungsergebnisses beiträgt.

Gegenstand der Erfindung ist daher die Verwendung von Morpholinderivaten der allgemeinen Formel (I), 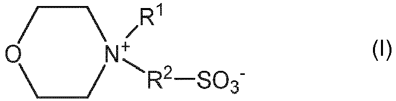 in der R¹ für einen linearen oder verzweigten Alkylrest mit 6 bis 20 C-Atomen, insbesondere 12 bis 16 C-Atomen und R² für einen linearen oder verzweigten Alkylenrest mit 2 bis 20 C-Atomen, insbesondere 3 bis 16 C-Atomen steht, zur Verbesserung der Reinigungsleistung von Wasch- oder Reinigungsmitteln gegenüber öl- und/oder fetthaltigen Anschmutzungen beim Waschen von Textilien oder Reinigen harter Oberflächen in insbesondere builderhaltiger wässriger Flüssigkeit. Das Mittel enthält vorzugsweise 0,05 Gew.-% bis 20 Gew.-%, insbesondere 0,1 Gew.-% bis 10 Gew.-% an Morpholinderivat der allgemeinen Formel (I). In bevorzugten Verbindungen der allgemeinen Formel (I) ist der Rest R² eine n-Propylengruppe oder eine n-Butylengruppe, wobei das N-Atom und der Substituent -SO₃⁻ sich an den entgegengesetzten endständigen Positionen befinden. In außerdem bevorzugten Verbindungen der allgemeinen Formel (I) ist der Rest R¹ eine lineare Alkylgruppe.

Morpholinderivate der allgemeinen Formel (I) können wie in Tenside Surf. Det. 50 (2013) 1, Seiten 45 bis 51 beschrieben hergestellt werden. Im Rahmen dieser Verwendung ist bevorzugt, ein ein solches Morpholinderivat enthaltendes Wasch- oder Reinigungsmittel einzusetzen, obwohl auch die separate Zugabe von Morpholinderivat der allgemeinen Formel (I) und eines davon freien Wasch- oder Reinigungsmittels zu einer zum Waschen von Wäsche oder zum Reinigen einer harten Oberfläche vorgesehenen Flüssigkeit, insbesondere Wasser, möglich ist.

Gewünschtenfalls können die erfindungsgemäßen Mittel noch weitere in Wasch- oder Reinigungsmitteln übliche Inhaltsstoffe enthalten, solange diese nicht in unzumutbarer Weise mit dem Morpholinderivat wechselwirken. Beispielhaft seien hier Builder, Bleichmittel, Enzyme, weitere Tenside, Lösungsmittel und Duftstoffe genannt.

Im Rahmen der erfindungsgemäßen Verwendung ist bevorzugt, wenn die Konzentration an Morpholinderivat der allgemeinen Formel (I) in der wässrigen Flotte, wie sie beispielsweise in Wasch- oder Geschirrspülmaschinen aber auch bei der Handwäsche, dem manuellen Geschirrspülen oder Reinigen anderer harter Oberflächen oder gegebenenfalls bei der Reinigung von Teppichen oder Polstermaterialien zum Einsatz kommt, 0,005 g/l bis 1 g/l, insbesondere 0,1 g/l bis 0,5 g/l beträgt. Die erfindungsgemäße Verwendung wird vorzugsweise bei Temperaturen im Bereich von 10 °C bis 95 °C, insbesondere 20 °C bis 40 °C durchgeführt. Die erfindungsgemäße Verwendung wird vorzugsweise bei pH-Werten im Bereich von pH 5 bis pH 12, insbesondere von pH 7 bis pH 11 durchgeführt.

Wasch- oder Reinigungsmittel, die als insbesondere pulverförmige Feststoffe, in nachverdichteter Teilchenform, als homogene Lösungen oder Suspensionen vorliegen können, können außer dem erfindungsgemäß zu verwendenden Morpholinderivat im Prinzip alle bekannten und in derartigen Mitteln üblichen Inhaltsstoffe enthalten. Die Mittel können insbesondere Buildersubstanzen, weitere oberflächenaktive Tenside, Wasser, wassermischbare organische Lösungsmittel, Enzyme, Sequestrierungsmittel, Elektrolyte, pH-Regulatoren, Polymere mit Spezialeffekten, wie soil release-Polymere, Farbübertragungsinhibitoren, Vergrauungsinhibitoren, knitterreduzierende und formerhaltende polymere Wirkstoffe, Additive zur Verbesserung des Ablauf- und Trocknungsverhaltens, Korrosionsinhibitoren und weitere Hilfsstoffe, wie optische Aufheller, Schaumregulatoren, Farb- und Duftstoffe enthalten.

Die Mittel können neben dem Morpholinderivat der allgemeinen Formel (I) ein oder mehrere weitere Tenside enthalten, wobei insbesondere anionische Tenside, nichtionische Tenside und deren Gemische in Frage kommen, aber auch kationische und/oder weitere amphotere Tenside enthalten sein können. Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen, insbesondere Natriumionen, als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen, die sogenannten Alkylsulfate, und die Sulfatierungsprodukte der genannten nichtionischen Tenside mit niedrigem Ethoxylierungsgrad, die sogenannten Ethersulfate. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Derartige weitere Tenside sind in Wasch- oder Reinigungsmitteln in Mengenanteilen von vorzugsweise 0,5 Gew.-% bis 15 Gew.-%, insbesondere von 1 Gew.-% bis 10 Gew.-%, enthalten, wobei alkoxylierte Alkohole, Alkylsulfate und Ethersulfate sowie Mischungen aus mindestens 2 dieser Tenside bevorzugt sind. Bevorzugt ist, wenn das Gewichtsverhältnis von Morpholinderivat der allgemeinen Formel (I) zu weiterem Tensid, insbesondere alkoxyliertem Alkohol und/oder Aniontensid, im Bereich von 1:30 bis 30:1, insbesondere von 50:50 bis 20:80 liegt. In ebenfalls bevorzugten Ausführungsformen der Erfindung ist kein nichtionisches Tensid vorhanden.

Mittel zum Einsatz in der Textilwäsche können als kationische Aktivsubstanzen mit textilweichmachender Wirkung insbesondere einen oder mehrere der kationischen, textilweichmachenden Stoffe der allgemeinen Formeln II, III oder IV enthalten: 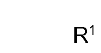 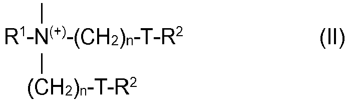 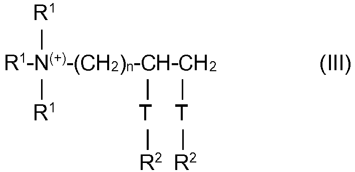 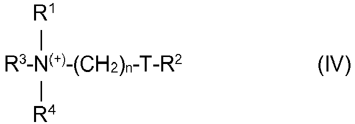 worin jede Gruppe R¹ unabhängig voneinander ausgewählt ist aus C₁₋₆-Alkyl-, -Alkenyl- oder -Hydroxyalkylgruppen; jede Gruppe R² unabhängig voneinander ausgewählt ist aus C₈₋₂₈-Alkyl- oder -Alkenylgruppen; R³ = R¹ oder (CH₂)ₙ-T-R²; R⁴ = R¹ oder R² oder (CH₂)ₙ-T-R²; T = -CH₂-, -O-CO- oder -CO-O- und n eine ganze Zahl von 0 bis 5 ist. Die kationischen textilweichmachenden Stoffe weisen übliche Anionen in zum Ladungsausgleich notwendiger Art und Anzahl auf, wobei diese neben beispielsweise Halogeniden auch aus den anionischen Tensiden ausgewählt werden können. In bevorzugten Ausführungsformen kommen Hydroxyalkyl-trialkyl-ammoniumverbindungen, insbesondere C₁₂₋₁₈-Alkyl(hydroxyethyl)dimethylammoniumverbindungen, und vorzugsweise deren Halogenide, insbesondere Chloride, zum Einsatz. Das Mittel enthält vorzugsweise bis zu 25 Gew.-%, insbesondere 0,5 Gew.-% bis 15 Gew.-% kationischen textilweichmachenden Stoff.

Ein Wasch- oder Reinigungsmittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden beziehungsweise Dextrinen zugänglichen Polycarboxylate, und/oder polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättigter Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/oder einem veresterten Vinylalkohol oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure, wobei Maleinsäure besonders bevorzugt ist, und/oder ein Derivat einer Allylsulfonsäure, die in 2-Stellung mit einem Alkyl- oder Arylrest substituiert ist, sein. Derartige Polymere weisen im Allgemeinen eine relative Molekülmasse zwischen 1 000 und 200 000 auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen Mitteln eingesetzt.

Als wasserlösliche anorganische Buildermaterialien kommen insbesondere polymere Alkaliphosphate, die in Form ihrer alkalischen neutralen oder sauren Natrium- oder Kaliumsalze vorliegen können, in Betracht. Beispiele hierfür sind Tetranatriumdiphosphat, Dinatriumdihydrogendiphosphat, Pentanatriumtriphosphat, sogenanntes Natriumhexametaphosphat sowie die entsprechenden Kaliumsalze beziehungsweise Gemische aus Natrium- und Kaliumsalzen. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Waschmitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das beispielsweise nach den Angaben der deutschen Patentschrift DE 24 12 837 bestimmt werden kann, liegt in der Regel im Bereich von 100 mg bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisitikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können eingesetzt werden. In einer weiteren bevorzugten Ausführungsform wird ein kristallines Natriumschichtsilikat mit einem Modul von 2 bis 3 eingesetzt, wie es n aus Sand und Soda hergestellt werden kann. Kristalline Natriumsilikate mit einem Modul im Bereich von 1,9 bis 3,5 werden in einer weiteren bevorzugten Ausführungsform eingesetzt. In einer bevorzugten Ausgestaltung setzt man ein granulares Compound aus Alkalisilikat und Alkalicarbonat ein, wie es zum Beispiel unter dem Namen Nabion® 15 im Handel erhältlich ist. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in Wasch- oder Reinigungsmitteln vorzugsweise in Mengen bis zu 60 Gew.-%, insbesondere von 5 Gew.-% bis 40 Gew.-%, enthalten.

In einer bevorzugten Ausgestaltung der Verwendung weist ein Mittel, insbesondere ein Textilwaschmittel, einen wasserlöslichen Builderblock auf. Durch die Verwendung des Begriffes "Builderblock" soll hierbei ausgedrückt werden, daß die Mittel keine weiteren Buildersubstanzen enthalten als solche, die wasserlöslich sind, das heißt sämtliche in dem Mittel enthaltenen Buildersubstanzen sind in dem so charakterisierten "Block" zusammengefasst, wobei allenfalls die Mengen an Stoffen ausgenommen sind, die als Verunreinigungen beziehungsweise stabilisierende Zusätze in geringen Mengen in den übrigen Inhaltsstoffen der Mittel handelsüblicher Weise enthalten sein können. Unter dem Begriff "wasserlöslich" soll dabei verstanden werden, daß sich der Builderblock bei der Konzentration, die sich durch die Einsatzmenge des ihn enthaltenden Mittels bei den üblichen Bedingungen ergibt, rückstandsfrei löst. Vorzugsweise sind mindestens 15 Gew.-% und bis zu 55 Gew.-%, insbesondere 25 Gew.-% bis 50 Gew.-% an wasserlöslichem Builderblock in den Mitteln enthalten. Dieser setzt sich vorzugsweise zusammen aus den Komponenten
a) 5 Gew.-% bis 35 Gew.-% Citronensäure, Alkalicitrat und/oder Alkalicarbonat, welches auch zumindest anteilig durch Alkalihydrogencarbonat ersetzt sein kann,
b) bis zu 10 Gew.-% Alkalisilikat mit einem Modul im Bereich von 1,8 bis 2,5,
c) bis zu 2 Gew.-% Phosphonsäure und/oder Alkaliphosphonat,
d) bis zu 50 Gew.-% Alkaliphosphat, und
e) bis zu 10 Gew.-% polymerem Polycarboxylat,
wobei die Mengenangaben sich auf das gesamte Mittel beziehen. Dies gilt auch für alle folgenden Mengenangaben, sofern nicht ausdrücklich anders angegeben.

In einer bevorzugten Ausführungsform enthält der wasserlösliche Builderblock mindestens 2 der Komponenten b), c), d) und e) in Mengen größer 0 Gew.-%.

Hinsichtlich der Komponente a) sind in einer bevorzugten Ausführungsform 15 Gew.-% bis 25 Gew.-% Alkalicarbonat, welches zumindest anteilig durch Alkalihydrogencarbonat ersetzt sein kann, und bis zu 5 Gew.-%, insbesondere 0,5 Gew.-% bis 2,5 Gew.-% Citronensäure und/oder Alkalicitrat enthalten. In einer alternativen Ausführungsform sind als Komponente a) 5 Gew.-% bis 25 Gew.-%, insbesondere 5 Gew.-% bis 15 Gew.-% Citronensäure und/oder Alkalicitrat und bis zu 5 Gew.-% , insbesondere 1 Gew.-% bis 5 Gew.-% Alkalicarbonat, welches zumindest anteilig durch Alkalihydrogencarbonat ersetzt sein kann, enthalten. Falls sowohl Alkalicarbonat wie auch Alkalihydrogencarbonat vorhanden sind, weist die Komponente a) Alkalicarbonat und Alkalihydrogencarbonat vorzugsweise im Gewichtsverhältnis von 10:1 bis 1:1 auf.

Hinsichtlich der Komponente b) sind in einer bevorzugten Ausführungsform 1 Gew.-% bis 5 Gew.-% Alkalisilikat mit einem Modul im Bereich von 1,8 bis 2,5 enthalten.

Hinsichtlich der Komponente c) sind in einer bevorzugten Ausführungsform 0,05 Gew.-% bis 1 Gew.-% Phosphonsäure und/oder Alkaliphosphonat enthalten. Unter Phosphonsäuren werden dabei auch gegebenenfalls substituierte Alkylphosphonsäuren verstanden, die auch mehrere Phosphonsäuregruppierungen aufweisen könne (sogenannte Polyphosphonsäuren). Bevorzugt werden sie ausgewählt aus den Hydroxy- und/oder Aminoalkylphosphonsäuren und/oder deren Alkalisalzen, wie zum Beispiel Dimethylaminomethandiphosphonsäure, 3-Aminopropan-1-hydroxy-1,1-diphosphonsäure, 1-Amino-1-phenyl-methandiphosphonsäure, 1-Hydroxyethan-1,1-diphosphonsäure, Amino-tris(methylenphosphonsäure), N,N,N',N'-Ethylendiamin-tetrakis(methylenphosphonsäure) und acylierte Derivate der phosphorigen Säure, die auch in beliebigen Mischungen eingesetzt werden können.

Hinsichtlich der Komponente d) sind in einer bevorzugten Ausführungsform 15 Gew.-% bis 35 Gew.-% Alkaliphosphat, insbesondere Trinatriumpolyphosphat, enthalten. Alkaliphosphat ist dabei die summarische Bezeichnung für die Alkalimetall- (insbesondere Natrium- und Kalium-) - Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei. Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Madrellsches Salz übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° (Zersetzung unter Bildung von (KPO₃)ₓ, Kaliumpolyphosphat) und ist leicht löslich in Wasser. Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol (Dichte 2,066 gcm⁻³, Wasserverlust bei 95°), 7 Mol (Dichte 1,68 gcm⁻³, Schmelzpunkt 48° unter Verlust von 5 H₂O) und 12 Mol Wasser (Dichte 1,52 gcm⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres oder zweibasiges Kaliumphosphat), K₂HPO₄, ist ein amorphes, weißes Salz, das in Wasser leicht löslich ist. Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit alkalischer Reaktion leicht löslich. Es entsteht z.B. beim Erhitzen von Thomasschlacke mit Kohle und Kaliumsulfat. Trotz des höheren Preises werden die leichter löslichen, daher hochwirksamen, Kaliumphosphate gegenüber entsprechenden Natrium-Verbindungen vielfach bevorzugt. Tetranatriumdiphosphat (Natriumpyrophosphat), Na₄P₂O₇, existiert in wasserfreier Form (Dichte 2,534 gcm⁻³, Schmelzpunkt 988°, auch 880° angegeben) und als Decahydrat (Dichte 1,815-1,836 gcm⁻³, Schmelzpunkt 94° unter Wasserverlust). Bei Substanzen sind farblose, in Wasser mit alkalischer Reaktion lösliche Kristalle. Na₄P₂O₇ entsteht beim Erhitzen von Dinatriumphosphat auf >200° oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt. Durch Kondensation des NaH₂PO₄ bzw. des KH₂PO₄ entstehen höhermolekulare Natrium- und Kaliumphosphate, bei denen man cyclische Vertreter, die Natrium- bzw. Kaliummetaphosphate und kettenförmige Typen, die Natrium- bzw. Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Madrellsches Salz. Alle höheren Natrium- und Kaliumphosphate werden gemeinsam als kondensierte Phosphate bezeichnet. Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung, durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind einsetzbar.

Hinsichtlich der Komponente e) sind in einer bevorzugten Ausführungsform der Mittel 1,5 Gew.-% bis 5 Gew.-% polymeres Polycarboxylat, insbesondere ausgewählt aus den Polymerisations- beziehungsweise Copolymerisationsprodukten von Acrylsäure, Methacrylsäure und/oder Maleinsäure enthalten. Unter diesen sind die Homopolymere der Acrylsäure und unter diesen wiederum solche mit einer mittleren Molmasse im Bereich von 5 000 D bis 15 000 D (PA-Standard) besonders bevorzugt.

Als in den Mitteln verwendbare Enzyme kommen solche aus der Klasse der Proteasen, Lipasen, Cutinasen, Amylasen, Pullulanasen, Mannanasen, Cellulasen, Hemicellulasen, Xylanasen und Peroxidasen sowie deren Gemische in Frage, beispielsweise Proteasen wie Serin-Proteasen, insbesondere Subtilasen, besonders bevorzugt Subtilisine, die ein Wildtypenzym oder eine Subtilisin-Variante sein kann, wobei das Wildtypenzym oder das Ausgangsenzym der Variante vorzugsweise ausgewählt ist aus der Alkalischen Protease aus Bacillus amyloliquefaciens (BPN'), der Alkalischen Protease aus Bacillus licheniformis (Subtilisin Carlsberg), der Alkalischen Protease PB92, Subtilisin 147 und/oder 309 (Savinase®), der Alkalischen Protease aus Bacillus lentus, vorzugsweise aus Bacillus lentus (DSM 5483), der Alkalischen Protease aus Bacillus alcalophilus (DSM 11233), der Alkalischen Protease aus Bacillus gibsonii (DSM 14391) oder einer hierzu mindestens zu 70% identischen Alkalischen Protease, der Alkalischen Protease aus Bacillus sp. (DSM 14390) oder einer hierzu mindestens zu 98,5% identischen Alkalischen Protease, und der Alkalischen Protease aus Bacillus sp. (DSM 14392) oder einer hierzu mindestens zu 98,1 % identischen Alkalischen Protease, Amylasen wie Termamyl®, Amylase-LT®, Maxamyl®, Duramyl® und/oder Purafect® OxAm, Lipasen wie Lipolase®, Lipomax®, Lumafast®, Lipozym® und/oder Lipex®, Cellulasen wie Celluzyme® und/oder Carezyme®. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in Wasch- oder Reinigungsmitteln vorzugsweise in Mengen bis zu 10 Gew.-%, insbesondere von 0,2 Gew.-% bis 2 Gew.-%, enthalten.

In einer bevorzugten Ausführungsform enthält das Mittel 5 Gew.-% bis 50 Gew.-%, insbesondere 8 bis 30 Gew.-% anionisches und/oder nichtionisches Tensid, bis zu 60 Gew.-%, insbesondere 5 bis 40 Gew.-% Buildersubstanz und 0,2 Gew.-% bis 2 Gew.-% Enzym, ausgewählt aus den Lipasen, Cutinasen, Amylasen, Pullulanasen, Mannanasen, Cellulasen, Oxidasen und Peroxidasen sowie deren Gemischen.

Zu den in den Wasch- oder Reinigungsmitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den Mitteln vorzugsweise in Mengen nicht über 30 Gew.-%, insbesondere von 6 Gew.-% bis 20 Gew.-%, vorhanden.

Aus der Natur stammende Polymere, die in wässrigen flüssigen Mitteln als Verdickungsmittel Verwendung finden können, sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein, Cellulosederivate wie Carboxymethylcellulose, Hydroxyethyl- und -propylcellulose, und polymere Polysaccharid-Verdickungsmittel wie Xanthan; daneben kommen auch vollsynthetische Polymere wie Polyacryl- und Polymethacryl-Verbindungen, Vinylpolymere, Polycarbonsäuren, Polyether, Polyimine, Polyamide und Polyurethane als Verdicker in Frage.

Zur Einstellung eines gewünschten, sich durch die Mischung der übrigen Komponenten nicht von selbst ergebenden pH-Werts können die Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Mitteln vorzugsweise nicht über 20 Gew.-%, insbesondere von 1,2 Gew.-% bis 17 Gew.-%, enthalten.

Schmutzablösevermögende Polymere, die oft als "Soil Release"-Wirkstoffe oder wegen ihres Vermögens, die behandelte Oberfläche, zum Beispiel der Faser, schmutzabstoßend auszurüsten, als "Soil Repellents" bezeichnet werden, sind beispielsweise nichtionische oder kationische Cellulosederivate. Zu den insbesondere polyesteraktiven schmutzablösevermögenden Polymeren gehören Copolyester aus Dicarbonsäuren, beispielsweise Adipinsäure, Phthalsäure oder Terephthalsäure, Diolen, beispielsweise Ethylenglykol oder Propylenglykol, und Polydiolen, beispielsweise Polyethylenglykol oder Polypropylenglykol. Zu den bevorzugt eingesetzten schmutzablösevermögenden Polyestern gehören solche Verbindungen, die formal durch Veresterung zweier Monomerteile zugänglich sind, wobei das erste Monomer eine Dicarbonsäure HOOC-Ph-COOH und das zweite Monomer ein Diol HO-(CHR¹¹-)ₐOH, das auch als polymeres Diol H-(O-(CHR¹¹-)ₐ)_{b}OH vorliegen kann, ist. Darin bedeutet Ph einen o-, m- oder p-Phenylenrest, der 1 bis 4 Substituenten, ausgewählt aus Alkylresten mit 1 bis 22 C-Atomen, Sulfonsäuregruppen, Carboxylgruppen und deren Mischungen, tragen kann, R¹¹ Wasserstoff, einen Alkylrest mit 1 bis 22 C-Atomen und deren Mischungen, a eine Zahl von 2 bis 6 und b eine Zahl von 1 bis 300. Vorzugsweise liegen in den aus diesen erhältlichen Polyestern sowohl Monomerdioleinheiten -O-(CHR¹¹-)ₐO- als auch Polymerdioleinheiten -(O-(CHR¹¹-)ₐ)_{b}O- vor. Das molare Verhältnis von Monomerdioleinheiten zu Polymerdioleinheiten beträgt vorzugsweise 100:1 bis 1:100, insbesondere 10:1 bis 1:10. In den Polymerdioleinheiten liegt der Polymerisationsgrad b vorzugsweise im Bereich von 4 bis 200, insbesondere von 12 bis 140. Das Molekulargewicht beziehungsweise das mittlere Molekulargewicht oder das Maximum der Molekulargewichtsverteilung bevorzugter schmutzablösevermögender Polyester liegt im Bereich von 250 bis 100 000, insbesondere von 500 bis 50 000. Die dem Rest Ph zugrundeliegende Säure wird vorzugsweise aus Terephthalsäure, Isophthalsäure, Phthalsäure, Trimellithsäure, Mellithsäure, den Isomeren der Sulfophthalsäure, Sulfoisophthalsäure und Sulfoterephthalsäure sowie deren Gemischen ausgewählt. Sofern deren Säuregruppen nicht Teil der Esterbindungen im Polymer sind, liegen sie vorzugsweise in Salzform, insbesondere als Alkali- oder Ammoniumsalz vor. Unter diesen sind die Natrium- und Kaliumsalze besonders bevorzugt. Gewünschtenfalls können statt des Monomers HOOC-Ph-COOH geringe Anteile, insbesondere nicht mehr als 10 Mol-% bezogen auf den Anteil an Ph mit der oben gegebenen Bedeutung, anderer Säuren, die mindestens zwei Carboxylgruppen aufweisen, im schmutzablösevermögenden Polyester enthalten sein. Zu diesen gehören beispielsweise Alkylen- und Alkenylendicarbonsäuren wie Malonsäure, Bernsteinsäure, Fumarsäure, Maleinsäure, Glutarsäure, Adipinsäure, Pimelinsäure, Korksäure, Azelainsäure und Sebacinsäure. Zu den bevorzugten Diolen HO-(CHR¹¹-)ₐOH gehören solche, in denen R¹¹ Wasserstoff und a eine Zahl von 2 bis 6 ist, und solche, in denen a den Wert 2 aufweist und R¹¹ unter Wasserstoff und den Alkylresten mit 1 bis 10, insbesondere 1 bis 3 C-Atomen ausgewählt wird. Unter den letztgenannten Diolen sind solche der Formel HO-CH₂-CHR¹¹-OH, in der R¹¹ die obengenannte Bedeutung besitzt, besonders bevorzugt. Beispiele für Diolkomponenten sind Ethylenglykol, 1,2-Propylenglykol, 1,3-Propylenglykol, 1,4-Butandiol, 1,5-Pentandiol, 1,6-Hexandiol, 1,8-Octandiol, 1,2-Decandiol, 1,2-Dodecandiol und Neopentylglykol. Besonders bevorzugt unter den polymeren Diolen ist Polyethylenglykol mit einer mittleren Molmasse im Bereich von 1000 bis 6000. Gewünschtenfalls können diese Polyester auch endgruppenverschlossen sein, wobei als Endgruppen Alkylgruppen mit 1 bis 22 C-Atomen und Ester von Monocarbonsäuren in Frage kommen. Den über Esterbindungen gebundenen Endgruppen können Alkyl-, Alkenyl- und Arylmonocarbonsäuren mit 5 bis 32 C-Atomen, insbesondere 5 bis 18 C-Atomen, zugrundeliegen. Zu diesen gehören Valeriansäure, Capronsäure, Önanthsäure, Caprylsäure, Pelargonsäure, Caprinsäure, Undecansäure, Undecensäure, Laurinsäure, Lauroleinsäure, Tridecansäure, Myristinsäure, Myristoleinsäure, Pentadecansäure, Palmitinsäure, Stearinsäure, Petroselinsäure, Petroselaidinsäure, Ölsäure, Linolsäure, Linolaidinsäure, Linolensäure, Eläostearinsäure, Arachinsäure, Gadoleinsäure, Arachidonsäure, Behensäure, Erucasäure, Brassidinsäure, Clupanodonsäure, Lignocerinsäure, Cerotinsäure, Melissinsäure, Benzoesäure, die 1 bis 5 Substituenten mit insgesamt bis zu 25 C-Atomen, insbesondere 1 bis 12 C-Atomen tragen kann, beispielsweise tert.-Butylbenzoesäure. Den Endgruppen können auch Hydroxymonocarbonsäuren mit 5 bis 22 C-Atomen zugrundeliegen, zu denen beispielsweise Hydroxyvaleriansäure, Hydroxycapronsäure, Ricinolsäure, deren Hydrierungsprodukt Hydroxystearinsäure sowie o-, m- und p-Hydroxybenzoesäure gehören. Die Hydroxymonocarbonsäuren können ihrerseits über ihre Hydroxylgruppe und ihre Carboxylgruppe miteinander verbunden sein und damit mehrfach in einer Endgruppe vorliegen. Vorzugsweise liegt die Anzahl der Hydroxymonocarbonsäureeinheiten pro Endgruppe, das heißt ihr Oligomerisierungsgrad, im Bereich von 1 bis 50, insbesondere von 1 bis 10. In einer bevorzugten Ausgestaltung der Erfindung werden Polymere aus Ethylenterephthalat und Polyethylenoxid-terephthalat, in denen die Polyethylenglykol-Einheiten Molgewichte von 750 bis 5000 aufweisen und das Molverhältnis von Ethylenterephthalat zu Polyethylenoxid-terephthalat 50:50 bis 90:10 beträgt, allein oder in Kombination mit Cellulosederivaten verwendet.

Zu den für den Einsatz in Mitteln für die Wäsche von Textilien in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol und gegebenenfalls weiteren Monomeren.

Die Mittel können Knitterschutzmittel enthalten, da textile Flächengebilde, insbesondere aus Reyon, Wolle, Baumwolle und deren Mischungen, zum Knittern neigen können, weil die Einzelfasern gegen Durchbiegen, Knicken, Pressen und Quetschen quer zur Faserrichtung empfindlich sind. Hierzu zählen beispielsweise synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Vergrauungsinhibitoren haben die Aufgabe, den von der harten Oberfläche und insbesondere von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Die Mittel können optische Aufheller, unter diesen insbesondere Derivate der Diaminostilbendisulfonsäure beziehungsweise deren Alkalimetallsalze, enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Waschverfahren kann es von Vorteil sein, den Mitteln übliche Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

Als in den Mitteln, insbesondere den Mitteln in fester Form, gegebenenfalls enthaltene Persauerstoffverbindungen kommen insbesondere organische Persäuren oder persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, wie Perborat, Percarbonat und/oder Persilikat, in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat, Alkaliperborat-Tetrahydrat oder Wasserstoffperoxid in Form wässriger Lösungen, die 3 Gew.-% bis 10 Gew.-% Wasserstoffperoxid enthalten, eingesetzt. Vorzugsweise sind Persauerstoffverbindungen in Mengen von bis zu 50 Gew.-%, insbesondere von 5 Gew.-% bis 30 Gew.-%, in Wasch- oder Reinigungsmitteln vorhanden.

Zusätzlich können übliche Bleichaktivatoren, die unter Perhydrolysebedingungen Peroxocarbonsäuren oder Peroxoimidsäuren bilden, und/oder übliche die Bleiche aktivierende Übergangsmetallkomplexe eingesetzt werden. Die fakultativ, insbesondere in Mengen von 0,5 Gew.-% bis 6 Gew.-%, vorhandene Komponente der Bleichaktivatoren umfasst die üblicherweise verwendeten N- oder O-Acylverbindungen, beispielsweise mehrfach acylierte Alkylendiamine, insbesondere Tetraacetylethylendiamin, acylierte Glykolurile, insbesondere Tetraacetylglykoluril, N-acylierte Hydantoine, Hydrazide, Triazole, Urazole, Diketopiperazine, Sulfurylamide und Cyanurate, außerdem Carbonsäureanhydride, insbesondere Phthalsäureanhydrid, Carbonsäureester, insbesondere Natrium-isononanoyl-phenolsulfonat, und acylierte Zuckerderivate, insbesondere Pentaacetylglukose, sowie kationische Nitrilderivate wie Trimethylammoniumacetonitril-Salze. Die Bleichaktivatoren können zur Vermeidung der Wechselwirkung mit den Perverbindungen bei der Lagerung in bekannter Weise mit Hüllsubstanzen überzogen beziehungsweise granuliert worden sein, wobei mit Hilfe von Carboxymethylcellulose granuliertes Tetraacetylethylendiamin mit mittleren Korngrößen von 0,01 mm bis 0,8 mm, granuliertes 1,5-Diacetyl-2,4-dioxohexahydro-1,3,5-triazin, und/oder in Teilchenform konfektioniertes Trialkylammoniumacetonitril besonders bevorzugt ist. In Wasch- oder Reinigungsmitteln sind derartige Bleichaktivatoren vorzugsweise in Mengen bis zu 8 Gew.-%, insbesondere von 2 Gew.-% bis 6 Gew.-%, jeweils bezogen auf gesamtes Mittel, enthalten.

In einer bevorzugten Ausführungsform der Verwendung ist ein Mittel flüssig und enthält einschließlich des Morpholinderivats bis zu 30 Gew.-%, insbesondere 2 Gew.-% bis 15 Gew.-% Tensid, bis zu 3 Gew.-%, insbesondere 0,05 Gew.-% bis 1 Gew.-% Verdicker, bis zu 10 Gew.-%, insbesondere 0,1 Gew.-% bis 3 Gew.-% Parfüm, bis zu 0,1 Gew.-% Farbstoff, bis zu 5 Gew.-%, insbesondere 0,5 Gew.-% bis 4,5 Gew.-% eines oder mehrerer Enzyme und auf 100 Gew.-% Wasser.

Die Herstellung fester Mittel bietet keine Schwierigkeiten und kann in im Prinzip bekannter Weise, zum Beispiel durch Sprühtrocknen oder Granulation, erfolgen. Zur Herstellung der Mittel mit erhöhtem Schüttgewicht, insbesondere im Bereich von 650 g/l bis 950 g/l, ist ein einen Extrusionsschritt aufweisendes Verfahren bevorzugt. Wasch- oder Reinigungsmittel in Form wässriger oder sonstige übliche Lösungsmittel enthaltender Lösungen werden besonders vorteilhaft durch einfaches Mischen der Inhaltsstoffe, die in Substanz oder als Lösung in einen automatischen Mischer gegeben werden können, hergestellt. In einer bevorzugten Ausführung der Verwendung von Mitteln für das insbesondere maschinelle Waschen oder Reinigen sind diese tablettenförmig.

### Beispiele

### Beispiel 1:

**Tabelle 1: Waschmittelzusammensetzungen (Angaben in Gew %)**

| | A | B | C | D | E | F | G | H |
|---|---|---|---|---|---|---|---|---|
| C₉₋₁₃-Alkylbenzolsulfonat, Na-Salz | 9 | 10 | 6 | 7 | 5 | 15 | 15 | 9 |
| C₁₂₋₁₈-Fettalkohol mit 7 EO | 8 | 9 | 6 | 7 | 5 | 6 | 11 | 10 |
| C₁₂₋₁₄-Fettalkoholsulfat mit 2EO | - | - | 8 | 7 | 10 | 2 | 2 | 5 |
| C₁₂₋₁₈-Fettsäure, Na-Salz | 4 | 3 | 3 | 3 | 4 | 2 | 4 | 7 |
| erfindungswesentliches Morpholinderivat | 3 | 3 | 3 | 3 | 3 | 3 | 3 | 3 |
| Zitronensäure | 2 | 3 | 3 | 2 | 2 | 2 | 2 | 3 |
| Natriumhydroxid, 50 % | 3 | 3 | 2 | 3 | 3 | 3 | 3 | 4 |
| Borsäure | 1 | 1 | 1 | 1 | 1 | 1 | 1 | 1 |
| Enzyme (Amylase, Protease, Cellulase) | + | + | + | + | + | + | + | + |
| Parfüm | 1 | 0,5 | 0,5 | 1 | 1 | 1 | 1 | 1 |
| Glycerin | 3 | 2 | 2 | 2 | 2 | - | - | 2 |
| Propandiol | - | - | - | - | - | 5 | 5 | - |
| Ethanol | 1,5 | 1,5 | 1,5 | 1,5 | 1,5 | 1,5 | 1,5 | 5 |
| PVA/Maleinsäure-Copolymer | 0,1 | - | 0,1 | - | - | - | - | - |
| Optischer Aufheller | - | 0,1 | - | 0,1 | 0,2 | 0,2 | 0,2 | 0,2 |
| Alkylaminophosphonsäure | 0,5 | 0,5 | 0,5 | 0,5 | 0,5 | 0,5 | 0,5 | 0,5 |
| Wasser | auf 100 | | | | | | | |

### Beispiel 2: Waschtest

Verwendet wurden die ansonsten identisch zusammengesetzten wasserhaltigen Flüssigwaschmittel V1 mit 6 Gew.-% Na-C₁₂₋₁₄-Alkyl-7EO-sulfat und E1 bis E3 mit stattdessen 3 Gew.-% des in der nachfolgenden Tabelle 2 angegebenen Morpholinderivats und 3 Gew.-% Na-C₁₂₋₁₄-Alkyl-7EO-sulfat. Standardisierte Anschmutzungen auf Baumwolle wurden mit einem dieser Mittel bei 40 °C gewaschen, dann gespült, getrocknet und anschließend ihr Remissionswert spektralphotometrisch (Minolta® CR400-1) vermessen (Waschzeit 1h, Waschmitteldosierung 4,12 g/l, 5-fach Bestimmung des Helligkeitswerts Y). Die in Tabelle 2 angegebenen Werte zeigen die ΔY-Werte der Remissionsmessung bei Einsatz der Mittel E1 bis E3 im Vergleich zum Waschmittel V1, wobei höhere Werte eine bessere Auswaschbarkeit bedeuten. Folgende fettige Anschmutzungen wurden verwendet:
Rinderfett (I)
Schweineschmalz, gefärbt (II)
Palmfett, gefärbt (III)
Pigment/Hautfett (IV)

**Tabelle 2:**

| Mittel mit / Anschmutzung | I | II | III | IV |
|---|---|---|---|---|
| E1 3-(N-Dodecylmorpholino)-1-propansulfat | 2,6 | 1,6 | 1,4 | 6,4 |
| E2 3-(N-Tetradecylmorpholino)-1-propansulfat | 6,6 | 4,6 | 2,7 | 8,8 |
| E3 3-(N-Hexadecylmorpholino)-1-propansulfat | 6,3 | 3,5 | 3,6 | 5,8 |

## Patentansprüche

1. Verwendung von Morpholinderivaten der allgemeinen Formel (I), 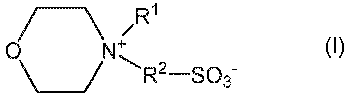 in der R¹ für einen linearen oder verzweigten Alkylrest mit 6 bis 20 C-Atomen und R² für einen linearen oder verzweigten Alkylenrest mit 2 bis 20 C-Atomen steht, zur Verbesserung der Reinigungsleistung von Wasch- oder Reinigungsmitteln gegenüber öl- und/oder fetthaltigen Anschmutzungen beim Waschen von Textilien oder Reinigen harter Oberflächen in insbesondere builderhaltiger wässriger Flüssigkeit.

2. Verwendung nach Anspruch 1, **dadurch gekennzeichnet, dass** man ein Mittel einsetzt, das 0,05 Gew.-% bis 20 Gew.-%, insbesondere 0,1 Gew.-% bis 10 Gew.-% an Morpholinderivat der allgemeinen Formel (I) enthält.

3. Verwendung nach Anspruch 1 oder 2, **dadurch gekennzeichnet, dass** man ein Mittel einsetzt, das weitere Tenside, insbesondere alkoxylierte Alkohole, Alkylsulfate und Ethersulfate sowie Mischungen aus mindestens 2 dieser Tenside, in Mengenanteilen von 0,5 Gew.-% bis 15 Gew.-%, insbesondere von 1 Gew.-% bis 10 Gew.-%, enthält.

4. Verwendung nach Anspruch 3, **dadurch gekennzeichnet, dass** das Gewichtsverhältnis von Morpholinderivat der allgemeinen Formel (I) zu weiterem Tensid, insbesondere alkoxyliertem Alkohol und/oder Aniontensid, im Bereich von 1:30 bis 30:1, insbesondere von 50:50 bis 20:80 liegt.

5. Verwendung nach einem der Ansprüche 1 bis 4, **dadurch gekennzeichnet, dass** man ein Mittel einsetzt, das flüssig ist und einschließlich des Morpholinderivats bis zu 30 Gew.-%, insbesondere 2 Gew.-% bis 15 Gew.-% Tensid, bis zu 3 Gew.-%, insbesondere 0,05 Gew.-% bis 1 Gew.-% Verdicker, bis zu 10 Gew.-%, insbesondere 0,1 Gew.-% bis 3 Gew.-% Parfüm, bis zu 0,1 Gew.-% Farbstoff, bis zu 5 Gew.-%, insbesondere 0,5 Gew.-% bis 4,5 Gew.-% eines oder mehrerer Enzyme und auf 100 Gew.-% Wasser enthält.

6. Verwendung nach einem der Ansprüche 1 bis 5, **dadurch gekennzeichnet, dass** die Konzentration an Morpholinderivat der allgemeinen Formel (I) in wässriger Flotte 0,005 g/l bis 1 g/l, insbesondere 0,01 g/l bis 0,5 g/l beträgt.

7. Verwendung nach einem der Ansprüche 1 bis 6, **dadurch gekennzeichnet, dass** im Morpholinderivat der allgemeinen Formel (I) R¹ für einen Alkylrest mit 12 bis 16 C-Atomen steht

8. Verwendung nach einem der Ansprüche 1 bis 7, **dadurch gekennzeichnet, dass** im Morpholinderivat der allgemeinen Formel (I) R² für einen Alkylenrest mit 3 bis 16 C-Atomen steht.

9. Verwendung nach einem der Ansprüche 1 bis 8, **dadurch gekennzeichnet, dass** im Morpholinderivat der allgemeinen Formel (I) der Rest R² eine n-Propylengruppe oder eine n-Butylengruppe ist, wobei das N-Atom und der Substituent -SO₃⁻ sich an den entgegengesetzten endständigen Positionen befinden.

10. Verwendung nach einem der Ansprüche 1 bis 9, **dadurch gekennzeichnet, dass** im Morpholinderivat der allgemeinen Formel (I) der Rest R¹ eine lineare Alkylgruppe ist.

## Claims

1. The use of morpholine derivatives of general formula (I), 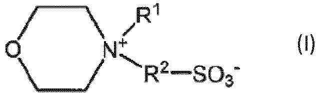 in which R¹ represents a linear or branched alkyl functional group having 6 to 20 C atoms and R² represents a linear or branched alkylene functional group having 2 to 20 C atoms, to improve the cleaning performance of washing or cleaning agents against oil- and/or fat-containing stains when washing textiles or cleaning hard surfaces in liquid that in particular contains builders and is aqueous.

2. The use according to claim 1, **characterized in that** an agent is used which contains 0.05 wt.% to 20 wt.%, in particular 0.1 wt.% to 10 wt.%, of morpholine derivative of the general formula (I).

3. The use according to claim 1 or 2, **characterized in that** an agent is used which contains further surfactants, in particular alkoxylated alcohols, alkyl sulfates and ether sulfates, and mixtures of at least 2 of these surfactants, in proportions of from 0.5 wt.% to 15 wt.%, in particular from 1 wt.% to 10 wt.%.

4. The use according to claim 3, **characterized in that** the weight ratio of morpholine derivative of the general formula (I) to further surfactant, in particular alkoxylated alcohol and/or anionic surfactant, is in the range of from 1:30 to 30:1, in particular from 50:50 to 20:80.

5. The use according to one of claims 1 to 4, **characterized in that** an agent is used which is liquid and, including the morpholine derivative, contains up to 30 wt.%, in particular 2 wt.% to 15 wt.%, surfactant, up to 3 wt.%, in particular 0.05 wt.% to 1 wt.%, thickener, up to 10 wt.%, in particular 0.1 wt.% to 3 wt.%, perfume, up to 0.1 wt.% dye, up to 5 wt.%, in particular 0.5 wt.% to 4.5 wt.%, of one or more enzymes and water up to 100 wt.%.

6. The use according to one of claims 1 to 5, **characterized in that** the concentration of morpholine derivative of the general formula (I) in an aqueous liquor is 0.005 g/l to 1 g/l, in particular 0.01 g/l to 0.5 g/l.

7. The use according to one of claims 1 to 6, **characterized in that** in the morpholine derivative of the general formula (I), R¹ represents an alkyl functional group having 12 to 16 C atoms.

8. The use according to one of claims 1 to 7, **characterized in that** in the morpholine derivative of the general formula (I), R² represents an alkylene functional group having 3 to 16 C atoms.

9. The use according to one of claims 1 to 8, **characterized in that** in the morpholine derivative of the general formula (I), the functional group R² is an n-propylene group or an n-butylene group, the N atom and the substituent -SO₃- being at the opposite terminal positions.

10. The use according to one of claims 1 to 9, **characterized in that** in the morpholine derivative of the general formula (I), the functional group R¹ is a linear alkyl group.

## Revendications

1. Utilisation de dérivés morpholiniques de formule générale (I), 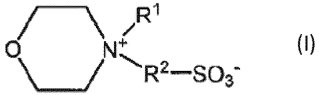 dans laquelle R¹ représente un radical alkyle linéaire ou ramifié ayant 6 à 20 atomes de carbone et R² représente un radical alkylène linéaire ou ramifié ayant 2 à 20 atomes de carbone, pour améliorer la performance de nettoyage des détergents ou agents de nettoyage par rapport aux salissures contenant des huiles et/ou des graisses lors du lavage de textiles ou du nettoyage de surfaces dures, en particulier de liquides aqueux contenant un adjuvant.

2. Utilisation selon la revendication 1, **caractérisée en ce que** l'on utilise un agent qui contient 0,05 % en poids à 20 % en poids, en particulier 0,1 % en poids à 10 % en poids d'un dérivé morpholinique de formule générale (I).

3. Utilisation selon la revendication 1 ou 2, **caractérisée en ce que** l'on utilise un agent qui contient d'autres agents tensioactifs, en particulier des alcools alcoxylés, des alkylsulfates et des éthersulfates, et des mélanges d'au moins 2 de ces agents tensioactifs, dans des proportions de 0,5 % à 15 % en poids, en particulier de 1 % à 10 % en poids.

4. Utilisation selon la revendication 3, **caractérisée en ce que** le rapport en poids du dérivé morpholinique de formule générale (I) à un autre agent tensioactif, en particulier un alcool alcoxylé et/ou un agent tensioactif anionique, est compris entre 1:30 et 30:1, en particulier entre 50:50 et 20:80.

5. Utilisation selon l'une quelconque des revendications 1 à 4, **caractérisée en ce que** l'on utilise un agent liquide, y compris le dérivé morphilinique, qui contient jusqu'à 30 % en poids, en particulier 2 % en poids à 15 % en poids d'agent tensioactif, jusqu'à 3 % en poids, en particulier 0,05 % en poids à 1 % en poids d'épaississant, jusqu'à 10 % en poids, en particulier 0,1 % en poids à 3 % en poids de parfum, jusqu'à 0,1 % en poids de colorant, jusqu'à 5 % en poids, en particulier 0,5 à 4,5 % en poids d'une ou plusieurs enzymes pour 100 % en poids d'eau.

6. Utilisation selon l'une quelconque des revendications 1 à 5, **caractérisée en ce que** la concentration du dérivé morpholinique de formule générale (l) dans le bain aqueux est de 0,005 g/l à 1 g/l, en particulier de 0,01 g/l à 0,5 g/l.

7. Utilisation selon l'une quelconque des revendications 1 à 6, **caractérisée en ce que** dans le dérivé morpholinique de formule générale (I), R¹ représente un radical alkyle ayant 12 à 16 atomes de carbone.

8. Utilisation selon l'une quelconque des revendications 1 à 7, **caractérisée en ce que** dans le dérivé morpholinique de formule générale (I), R² représente un radical alkylène ayant 3 à 16 atomes de carbone.

9. Utilisation selon l'une quelconque des revendications 1 à 8, **caractérisée en ce que** dans le dérivé morpholinique de formule générale (I), R² représente un groupe n-propylène ou un groupe n-butylène, l'atome d'azote et le substituant -SO₃- étant situés aux positions terminales opposées.

10. Utilisation selon l'une quelconque des revendications 1 à 9, **caractérisée en ce que** dans le dérivé morpholinique de formule générale (I), R¹ représente un groupe alkyle linéaire.
